(19) Europäisches Patentamt
European Patent Office
Office européen des brevets

(11) Veröffentlichungsnummer: **0 208 951 B1**

(12) **EUROPÄISCHE PATENTSCHRIFT**

(45) Veröffentlichungstag der Patentschrift: **16.08.89**

(51) Int. Cl.⁴: **A 61 K 7/06,** A 61 K 7/11

(21) Anmeldenummer: **86108496.0**

(22) Anmeldetag: **21.06.86**

(54) **Mittel zur Pflege des Haares.**

(30) Priorität: **06.07.85 DE 3524263**

(43) Veröffentlichungstag der Anmeldung: **21.01.87 Patentblatt 87/4**

(45) Bekanntmachung des Hinweises auf die Patenterteilung: **16.08.89 Patentblatt 89/33**

(84) Benannte Vertragsstaaten: **AT BE CH DE FR GB IT LI LU NL SE**

(56) Entgegenhaltungen:
**WO-A-85/02999**
**FR-A-2 383 660**
**FR-A-2 393 573**
**GB-A-2 063 671**
**US-A-3 954 960**
**US-A-4 391 286**

**PATENTS ABSTRACTS OF JAPAN, Band 2, Nr. 72 (C-78)[837], 31. Mai 1978; & JP-A-53 29 941 (MATSUSHITA DENKO K.K.) 20.03.1978**

(73) Patentinhaber: **Wella Aktiengesellschaft, Berliner Allee 65, D-6100 Darmstadt (DE)**

(72) Erfinder: **Gross, Paul, Forstweg 54, D-6100 Darmstadt (DE)**
Erfinder: **Wiegand, Udo, Stresemannstrasse 10, D-6100 Darmstadt 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 208 951 B1

ACTORUM AG

**Beschreibung**

Gegenstand der Erfindung ist ein Mittel zur Pflege des Haares, das nicht ausgespült werden muß, welches Tetraoxyethylenlaurylether und ein quaternisiertes Copolymerisat aus Vinylpyrrolidon mit Dimethylaminoethylmethacrylat (80:20) enthält.

Haarkurmittel dienen der Verbesserung der Haarkondition und sind üblicherweise Öl-in-Wasser-Emulsionen, die als Gerüstbestandteile ölige, halbfeste oder feste Substanzen, wie beispielsweise Paraffinöl, Vaseline, Wollfett, Wollfettalkohole, Fettsäureester und Kohlenwasserstoffwachse enthalten. Als Emulgatoren für solche Emulsionen werden normalerweise quaternäre Ammoniumverbindungen wie oxyethylierte Alkylammoniumphosphate, Alkyltrimethylammoniumchloride, Dialkyldimethylammoniumchloride, Alkyldimethylbenzylammoniumchloride und Alkylpyridiniumchloride, allein oder in Kombination mit nichtionogenen Emulgatoren, verwendet. Auch wäßrige Lösungen und wäßrige Gele mit einem Gehalt an quaternären Ammoniumverbindungen finden häufig als Haarkurmittel Anwendung.

Die vorstehend genannten monomeren quaternären Verbindungen dienen gleichzeitig zur Verbesserung der Naßkämmbarkeit und des Griffes, insbesondere des geschädigten Haares.

Derartige Zusätze quaternärer Ammoniumverbindungen verschlechtern jedoch die physiologische Verträglichkeit solcher Präparate, insbesondere die Augenverträglichkeit.

Demgegenüber wurde nun gefunden, daß Mittel zur Pflege des Haares auf der Basis einer wäßrigen, alkoholischen oder wäßrig-alkoholischen Lösung eines quaternisierten Copolymerisates und eines oxyethylierten Fettalkohols sowie gegebenenfalls Treibmitteln und üblichen Zusätzen dadurch gekennzeichnet, daß sie

a) als filmbildendes Harz 0,01 bis 0,09 Gew.-% eines quaternisierten Copolymerisates aus Vinylpyrrolidon und Dimethylaminoethylmethacrylat (80:20) und

b) als oxyethylierten Fettalkohol 0,01 bis 0,5 Gew.-% Tetraoxyethylenlaurylether

enthalten, eine überraschende und ausgezeichnete Verbesserung der Kämmbarkeit des Haares bewirken.

Das unter a) genannte Copolymerisat aus Vinylpyrrolidon und Dimethylaminoethylmethacrylat soll vorzugsweise mit Dimethylsulfat oder Diethylsulfat quaternisiert sein. Als quaternisiertes Copolymerisat aus Vinylpyrrolidon und Dimethylaminoethylmethacrylat (80:20) sind besonders die Handelsprodukte GAFQUAT® 734 und GAFQUAT® 755 der Firma GAF Corporation, New York geeignet. Diese weisen die folgende Formel auf:

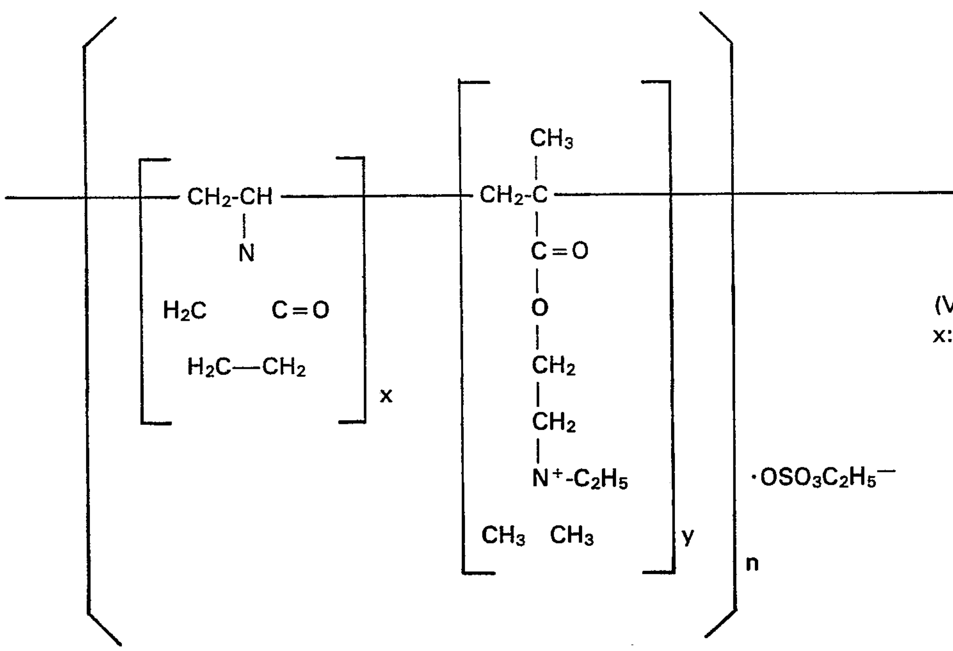

(Verhältnis x:y = 80:20)

Das mittlere Molekulargewicht beträgt bei GAFQUAT® 755 N etwa 1 000 000 (g/Mol) und bei GAFQUAT® 734 etwa 100 000 (g/Mol).

Tetraoxyethylenlaurylether der Formel

$$CH_3(CH_2)_{10}CH_2(OCH_2CH_2)_4OH$$

ist der Tetraoxyethylenether des Laurylalkohols.

In dem erfindungsgemäßen Mittel soll der Tetraoxyethylenlaurylether in einer Menge von 0,01 bis 0,5 Gew.-%, vorzugsweise in einer Menge von 0,03 bis 0,09 Gew.-%, enthalten sein. Der Gehalt an dem quaternisierten Copolymerisat aus Vinylpyrrolidon und Dimethylaminoethylmethacrylat beträgt 0,01 bis 0,09 Gew.-%, vorzugsweise 0,03 bis 0,07 Gew.-%. Ethylalkohol und Isopropylalkohol oder Gemische dieser beiden Alkohole können in dem erfindungsgemäßen Mittel in einer Menge von 0 bis 99,98 Gew.-% enthalten sein. Selbstverständlich können auch übliche haarkosmetische Zusätze wie beispielsweise Parfümöle, Kräuterextrakte, bakterizide oder fungizide Stoffe, Antischuppenmittel, Lösungsvermittler für Parfümöle, in dem erfindungsgemäßen Mittel enthalten sein, soweit solche Zusätze nützlich und zweckmäßig erscheinen. Weiterhin können auch Anfärbefarbstoffe zur Anfärbung des Präparates oder direkt auf das Haar aufziehende Farbstoffe zur gleichzeitigen Tönung des Haares enthalten sein.

Von letzteren Farbstoffen, die einzeln oder in Mischung vorliegen können, seien beispielsweise die folgenden Klassen erwähnt:

Aromatische Nitrofarbstoffe, z.B. 1,4-Diamino-2--nitrobenzol, Azofarbstoffe, z.B. Acid Brown 4 (C.I. Nr. 14 805), Anthrachinonfarbstoffe, z.B. Disperse Violet 4 (C.I. Nr. 61 105) und Triphenylmethanfarbstoffe, z.B. Basic Violet 1 (C.I. Nr. 42 535), wobei die Farbstoffe dieser Klassen je nach der Art ihrer Substituenten sauren, nichtionogenen oder basischen Charakter haben können. Ihre Gesamtkonzentration beträgt üblicherweise etwa 0,05 bis 2,0 Gew.-%.

Die erfindungsgemäßen Mittel können auch unter Zusatz eines Treibmittels in einem Druckbehälter abgefüllt vorliegen, wobei das Präparat als Schaum austritt und mittels eines mit einer Auftragedüse versehenen Ventils leicht dosiert und bequem auf dem Haar verteilt werden kann. Als Treibmittel sind beispielsweise leicht flüchtige Fluorchlorkohlenwasserstoffe, wie z.B. Difluordichlormethan, Trichlormonofluormethan, Tetrafluordichlorethan oder niedere Alkane, wie z.B. n-Butan und Propan oder auch Dimethylether sowie ferner bei den in Betracht kommenden Drücken gasförmig vorliegende Treibmittel, wie $N_2$, $N_2O$ und $CO_2$, geeignet. Die Treibmittel sind in diesen Mitteln zweckmäßigerweise in einer Menge von etwa 2 bis 10 Gew.-% enthalten.

Das erfindungsgemäße Mittel wird, üblicherweise nach der Haarwäsche, in dem handtuchtrockenen Haar in einer Menge von etwa 5 bis 15 g je nach Haarfülle verteilt. Anschließend wird das Haar durchgekämmt und sodann getrocknet.

Üblicherweise müssen Haarkurmittel, welche in Emulsionsform oder in Form von wäßrigen Gelen vorliegen, nach einer Einwirkungszeit von mehreren Minuten mit Wasser wieder ausgespült werden. Verblieben sie im Haar, wäre das Haar klebrig und daher für eine Frisurengestaltung unbrauchbar. Es ist deshalb ein besonderer Vorzug des erfindungsgemäßen Mittels, daß es ohne auszuspülen im Haar verbleiben kann. Der Anwender erspart sich dadurch die Zeit und Mühe des Ausspülens.

Die beobachtete ausgezeichnete Verbesserung der Kämmbarkeit des Haares ist überraschend und nur synergistisch erklärbar, da der Tetraoxyethylenlaurylether oder das quaternisierte Copolymerisat aus Vinylpyrrolidon und Dimethylaminoethylmethacrylat in entsprechender Lösung allein auf das Haar aufgebracht (siehe Testbeispiele C und D) nur eine mäßige und unbefriedigende Verbesserung der dort genannten Kriterien bewirkt.

Weiterhin wurde durch die nachstehenden Testbeispiele E, F, G und H nachgewiesen, daß andere Fettalkoholpolyoxyethylenether als Tetraoxyethylenlaurylether die für eine Verwendung in den erfindungsgemäßen Mitteln erforderliche synergistische Wirkung nicht aufweisen.

Bei dem erfindungsgemäßen Mittel zur Pflege des Haares kann auf Grund seiner ausgezeichneten kämmbarkeitsverbessernden Wirkung ganz auf die Verwendung quaternärer Ammoniumverbindungen verzichtet werden, es sei denn, man setzt sie dem Mittel in geringer Konzentration von etwa bis zu 0,2 Gew.-% als Konservierungsmittel zur Erzielung bakterizider oder fungizider Eigenschaften zu.

Die nachstehenden Beispiele sollen den Gegenstand der Erfindung näher erläutern.

*Beispiele*

*Beispiel 1*

Mittel in Form einer wäßrigen Lösung

| | |
|---|---|
| 0,08 g | Tetraoxyethylenlaurylether |
| 0,06 g | Copolymerisat aus Vinylpyrrolidon und Dimethylaminoethylmethacrylat im Verhältnis 80:20, quaternisiert mit Diethylsulfat |
| 99,86 g | Wasser, vollentsalzt |
| 100,0 g | |

*Beispiel 2*

Mittel in Form einer wäßrig-alkoholischen Lösung

| | |
|---|---|
| 0,06 g | Tetraoxyethylenlaurylether |
| 0,05 g | Copolymerisat aus Vinylpyrrolidon und Dimethylaminoethylmethacrylat im Verhältnis 80:20, quaternisiert mit Diethylsulfat |
| 15,00 g | Ethanol |
| 84,89 g | Wasser, vollentsalzt |
| 100,0 g | |

*Beispiel 3*

Mittel in Form einer Druckgaspackung

| | |
|---|---|
| 0,09 g | Tetraoxyethylenlaurylether |
| 0,07 g | Copolymerisat aus Vinylpyrrolidon und Dimethylaminoethylmethacrylat im Verhältnis 80:20, quaternisiert mit Diethylsulfat |
| 2,50 g | Isopropanol |
| 97,34 g | Wasser, vollentsalzt |
| 100,0 g | |

| | | |
|---|---|---|
| Abfüllung: | 96,0 g | Flüssigkeit der vorstehenden Zusammensetzung |
| | 2,8 g | Propan |
| | 1,2 g | Butan |

*Beispiel 4*

Mittel in Form einer Druckgaspackung

| | |
|---|---|
| 0,08 g | Tetraoxyethylenlaurylether |
| 0,06 g | Copolymerisat aus Vinylpyrrolidon und Dimethylaminoethylmethacrylat im Verhältnis 80:20, quaternisiert mit Diethylsulfat |
| 2,50 g | Ethanol |
| 97,36 g | Wasser, vollentsalzt |
| 100,0 g | |

Abfüllung: 96,0 Flüssigkeit der vorstehenden Zusammensetzung
2,0 Propan
1,6 g Butan
0,4 g Dimethylether

*Beispiel 5*

Mittel in Form einer wäßrig-alkoholischen Lösung

| | | |
|---|---|---|
| 0,09 | g | Tetraoxyethylenlaurylether |
| 0,07 | g | Copolymerisat aus Vinylpyrrolidon und Dimethylaminoethylmethacrylat im Verhältnis 80:20, quaternisiert mit Diethylsulfat |
| 0,03 | g | Basic Violet 1 (C.I. Nr. 42 535) |
| 2,50 | g | Ethanol |
| 97,31 | g | Wasser, vollentsalzt |
| 100,00 | g | |

Alle in der vorliegenden Anmeldung angegebenen Prozentzahlen stellen Gewichtsprozente dar.

*Versuchsbeispiele*

*Testbeispiel A*

Nach einer vorangegangenen Haarwäsche wurde das handtuchtrockene Haar von 15 Versuchspersonen mit einem Mittel gemäß Beispiel 1 behandelt. Fünf der Versuchspersonen hatten normales Haar. Die restlichen zehn Personen hatten geschädigtes bis stark geschädigtes Haar. Um eindeutige Ergebnisse zu erhalten und von Versuchsperson zu Versuchsperson abweichende Haarqualtäten auszuschalten, wurde das Haar in der Mitte gescheitelt und auf die eine Hälfte des Haares, in Abhängigkeir von der Haarfülle, 2,5 bis 7,5 g des Mittels aufgetragen, während die andere Hälfte unbehandelt blieb. Anschließend wurde jede Hälfte des Haares für sich durchgekämmt, danach getrocknet und dann das Haar frisiert. So konnte die Wirkung des Präparates von einer friseur-fachlichen Expertengruppe sicher beurteilt werden. Eine Bewertung erfolgte nach dem Schema für die zusammengefaßten Kriterien der Naßkämmbarkeit, Lockigkeit, Glanz, Griff und der statischen Aufladung des Haares:

Benotung: 1 = sehr gut
2 = gut
3 = ausreichend
4 = mangelhaft

Das Ergebnis des Versuches für die behandelte Haarhälfte im Vergleich zur unbehandelten Hälfte zeigt die nachstehende Tabelle 1.

TABELLE 1

| | Note 1 | Note 2 | Note 3 | Note 4 |
|---|---|---|---|---|
| Anzahl der Versuchspersonen | 12 | 2 | 1 | 0 |

*Testbeispiel B*

Die Haare einer weiteren Gruppe von 17 Versuchspersonen wurden mit einem Mittel gemäß Beispiel 3 in gleicher Weise wie im Testbeispiel A behandelt. Zehn Personen der Gruppe hatten geschädigtes bis stark geschädigtes Haar. Die restlichen 7 Personen hatten normales Haar. Die Ergebnisse dieses Tests sind in der nachstehenden Tabelle 2 zusammengefaßt.

TABELLE 2

| | Note 1 | Note 2 | Note 3 | Note 4 |
|---|---|---|---|---|
| Anzahl der Versuchspersonen | 13 | 3 | 1 | 0 |

*Testbeispiel C*

Zur Überprüfung des synergistischen Effektes wurden die Haare einer weiteren Testgruppe von 14 Personen, wie im Testbeispiel A beschrieben, halbseitig mit einer Zusammensetzung gemäß Beispiel 1 behandelt, jedoch wurde der Tetraoxyethylenlaurylether durch die entsprechende Menge an Wasser ersetzt. Die andere Hälfte des Haares blieb unbehandelt. 4 Versuchspersonen hatten normales Haar, und 10 Personen hatten geschädigtes Haar. Das Versuchsergebnis ist in Tabelle 3 wiedergegeben.

TABELLE 3

| | Note 1 | Note 2 | Note 3 | Note 4 |
|---|---|---|---|---|
| Anzahl der Versuchspersonen | 0 | 2 | 6 | 6 |

*Testbeispiel D*

Zur weiteren Überprüfung des synergistischen Effektes wurde eine vierte Testgruppe von 12 Personen, von denen 4 Personen normales und 8 Personen stark geschädigtes Haar hatten, in gleicher Weise wie in Testbeispiel A beschrieben, mit einer Zusammensetzung gemäß Beispiel 1 halbseitig behandelt, jedoch enthielt das Mittel kein quaternisiertes Copolymerisat aus Vinylpyrrolidon und Dimethylaminoethylmethacrylat (80:20). In der Zusammensetzung nach Beispiel 1 wurde dabei das quaternisierte Copolymerisat durch den entsprechenden Gewichtsanteil an Wasser ersetzt. Die zweite Hälfte des Haares blieb unbehandelt. Das Ergebnis des Versuches ist in der nachstehenden Tabelle 4 wiedergegeben.

TABELLE 4

| | Note 1 | Note 2 | Note 3 | Note 4 |
|---|---|---|---|---|
| Anzahl der Versuchspersonen | 0 | 3 | 3 | 6 |

Ein Vergleich der Tabellen 1 bzw. 2 sowohl mit der Tabelle 3 als auch mit der Tabelle 4 zeigt deutlich, daß die in den erfindungsgemäßen Mitteln enthaltene Kombination eine synergistische Wirkung im Vergleich zur Wirkung der Einzelkomponenten besitzt.

*Testbeispiel E*

Die Haare einer Testgruppe von 10 Personen mit geschädigtem bis stark geschädigtem Haar wurden analog Testbeispiel A halbseitig mit einer Präparatezusammensetzung nach Beispiel 1 behandelt, in dem jedoch der Tetraoxyethylenlaurylether mengengleich durch Trioxyethylenstearylether ersetzt worden war. Die andere Seite des Haares blieb unbehandelt. Das Versuchsergebnis zeigt die nachstehende Tabelle 5.

TABELLE 5

| | Note 1 | Note 2 | Note 3 | Note 4 |
|---|---|---|---|---|
| Anzahl der Versuchspersonen | 0 | 1 | 7 | 2 |

*Testbeispiel F*

Es wurden die Haare einer Gruppe von 9 Versuchspersonen mit geschädigtem bis stark geschädigtem Haar analog Testbeispiel A halbseitig mit einer Präparatezusammensetzung nach Beispiel 1 behandelt, in dem jedoch der Tetraoxyethylenlaurylether mengengleich durch Isostearylalkohol, ethoxyliert mit 10 Mol Ethylenoxid, ersetzt worden war. Die andere Seite Haares des blieb unbehandelt. Das Ergebnis des Versuches zeigt die nachstehende Tabelle 6.

TABELLE 6

| | Note 1 | Note 2 | Note 3 | Note 4 |
|---|---|---|---|---|
| Anzahl der Versuchspersonen | 0 | 1 | 3 | 5 |

Die vorstehenden Testbeispiele E und F zeigen, daß unter den Polyoxyethylenlaurylethern nur Tetraoxyethylenlaurylether einen deutlichen synergistischen Effekt aufweist.

*Testbeispiel G*

Eine weitere Gruppe von 10 Versuchspersonen mit geschädigtem bis stark geschädigtem Haar wurde analog Testbeispiel A halbseitig mit einer Zusammensetzung gemäß Beispiel 1 behandelt, in dem jedoch der Tetraoxyethylenlaurylether mengengleich durch Trioxyethylenlaurylether ersetzt worden war. Die andere Seite des Haares blieb unbehandelt. Das Ergebnis des Versuches zeigt die nachstehende Tabelle 7.

TABELLE 7

| | Note 1 | Note 2 | Note 3 | Note 4 |
|---|---|---|---|---|
| Anzahl der Versuchspersonen | 0 | 4 | 5 | 1 |

*Testbeispiel H*

Ebenso wurde eine Testgruppe von 10 Personen mit geschädigtem bis stark geschädigtem Haar analog Testbeispiel A halbseitig mit einer Zusammensetzung gemäß Beispiel 1 behandelt, in dem jedoch der Tetraoxyethylenlaurylether mengengleich durch Pentaoxyethylenlaurylether ersetzt worden war. Die andere Seite des Haares blieb unbehandelt.

Das Ergebnis zeigt die nachstehende Tabelle 8.

TABELLE 8

| | Note 1 | Note 2 | Note 3 | Note 4 |
|---|---|---|---|---|
| Anzahl der Versuchspersonen | 0 | 2 | 6 | 2 |

Die Resultate der vorstehenden Versuche zeigen, daß andere Fettalkoholpolyoxyethylenether als Tetraoxyethylenlaurylether für eine erfindungsgemäße Verwendung ungeeignet sind.

Die überraschende und ausgezeichnete synergistische Verbesserung der haarkonditionierenden Eigenschaften, insbesondere bei stark geschädigtem Haar, ist nach den beschriebenen Versuchsergebnissen auf die erfindungsgemäße Kombination von Tetraoxyethylenlaurylether mit einem quaternisierten Copolymerysat aus Vinylpyrrolidon und Dimethylaminoethylmethacrylat (80:20) beschränkt und ist mit anderen Fettalkoholpolyoxyethylenethern nichtzu erzielen.

**Patentansprüche**

**1. Mittel zur Pflege des Haares, das nicht ausgespült werden muß, auf der Basis einer wäßrigen, alkoholischen oder wäßrig-alkoholischen Lösung eines quaternisierten Copolymerisates und eines oxyethylierten Fettalkohols sowie gegebenenfalls Treibmitteln und üblichen Zusätzen, dadurch gekennzeichnet, daß es**

a) 0,01 bis 0,09 Gew.-% eines quaternisierten Copolymerisates aus Vinylpyrrolidon und Dimethylaminoethylmethacrylat (80:20) und

b) als oxyethylierten Fettalkohol 0,01 bis 0,5 Gew.-% Tetraoxyethylenlaurylether

enthält.

**2. Mittel nach Anspruch 1, dadurch gekennzeichnet, daß es 0,03 bis 0,07 Gew.-% eines mit**

Dimethyl- oder Diethylsulfat quaternisierten Copolymerisats aus Vinylpyrrolidon und Dimethylaminoethylmethacrylat (80:20) enthält.

3. Mittel nach Anspruch 1, dadurch gekennzeichnet, daß es 0,03 bis 0,09 Gew.-% Tetraoxyethylenlaurylether enthält.

4. Mittel nach Anspruch 1, dadurch gekennzeichnet, daß es zur gleichzeitigen Tönung des Haares 0,05 bis 2,0 Gew.-% eines direkt auf das Haar aufziehenden Farbstoffs enthält.

5. Mittel nach Anspruch 4, dadurch gekennzeichnet, daß der direkt auf das Haar aufziehende Farbstoff ausgewählt ist aus aromatischen Nitrofarbstoffen, Azofarbstoffen, Anthrachinonfarbstoffen und Triphenylmethanfarbstoffen.

6. Mittel nach den Ansprüchen 1 bis 5, dadurch gekennzeichnet, daß es 2 bis 10 Gew.-% eines Treibmittels enthält, des ausgewählt ist aus n-Butan, i-Butan, Propan, Difluordichlormethan, Trichlormonofluormethan, Tetrafluordichlorethan, Dimethylether, $N_2$, $N_2O$ und $CO_2$.

**Claims**

1. Hair care medium, which need not be rinsed out, based on an aqueous, alcoholic, or aqueous-alcoholic solution of a quaternarised copolymer and an oxyethylated fatty alcohol and, optionally, propellants and conventional additives, characterised in that it contains

a) 0.01 to 0.09 weight per cent of a quaternarised copolymer of vinyl pyrrolidone and dimethylaminoethylmethacrylate (80:20) and

b) 0.01 to 0.5 weight per cent of tetraoxyethylene laurylether as oxyethylated fatty alcohol.

2. Medium according to claim 1, characterised in that it contains 0.03 to 0.07 weight per cent of a copolymer of vinyl pyrrolidone and dimethylaminoethylmethacrylate (80:20) quarternised with dimethyl- or diethyl sulfate.

3. Medium according to claim 1, characterised in that it contains 0.03 to 0.09 weight per cent of tetraoxyethylene laurylether.

4. Medium according to claim 1, characterised in that it contains for the simultaneous toning of the hair, 0.05 to 2.0 weight per cent of a dye that is directly absorbed onto the hair.

5. Medium according to claim 4, characterised in that the dye that is directly absorbed onto the hair is selected from aromatic nitro-dyes, azo-dyes, anthraquinone dyes and triphenylmethane dyes.

6. Medium according to claim 1 to 5, characterised in that it contains 2 to 10 weight per cent of a propellant selected from n-butane, i-butane, propane, difluorodichloromethane, trichloromonofluoromethane, tetrafluorodichlorethane, dimethylether, $N_2$, $N_2O$ and $CO_2$.

**Revendications**

1. Produit pour soin des cheveux, qui n'a pas à être enlevé par rinçage, à base d'une solution aqueuse, alcoolique ou dans l'eau et l'alcool d'un produit de copolymérisation quaternisé et d'un alcool gras éthoxylé, ainsi éventuellement que d'agents moussants et d'additifs usuels, caractérisé en ce qu'il renferme

a) 0,01 à 0,9% en poids d'un produit de copolymérisation quaternisé de vinylpyrrolidone et de méthacrylate de diméthylaminoéthyle (80:20), et

b) comme alcool gras éthoxylé, 0,01 à 0,5% en poids de tétraoxyéthylène-lauryl-éther.

2. Produit selon la revendication 1, caractérisé en ce qu'il renferme 0,03 à 0,07% en poids d'un produit de copolymérisation de vinylpyrrolidone et de méthacrylate de diméthylaminoéthyle (80:20) quaternisé avec du diméthylsulfate ou diéthylsulfate.

3. Produit selon la revendication 1, caractérisé en ce qu'il renferme 0,03 a 0,09% en poids de tétraoxyéthylène-lauryléther.

4. Produit selon la revendication 1, caractérisé en ce qu'il renferme, pour le nuançage simultané des cheveux, 0,05 à 2,0% en poids d'un colorant à montée directe sur les cheveux.

5. Produit selon la revendication 4, caractérisé en ce que le colorant à montée directe sur les cheveux est choisi parmi des colorants aromatiques nitro, azoïques, anthrachinoniques et au triphénylméthane.

6. Produit selon les revendications 1 à 5, caractérisé en ce qu'il referme 2 à 10% en poids d'un agent moussant, chosi parmi le n-butane, l'isobutane, le propane, le difluorodichlorométhane, le trichloromonofluorométhane, le tétrafluorodichloroéthane, le diméthyléther, $N_2$, $N_2O$ et $CO_2$.